(19) **Europäisches Patentamt / European Patent Office / Office européen des brevets**

(11) **EP 4 640 142 A2**

(12) **EUROPEAN PATENT APPLICATION**

(43) Date of publication:
29.10.2025 Bulletin 2025/44

(21) Application number: 25201666.2

(22) Date of filing: 18.03.2022

(51) International Patent Classification (IPC):
**A61B 5/00** (2006.01)

(52) Cooperative Patent Classification (CPC):
**A61B 5/0077; A61B 5/02042; A61B 5/0261;
A61B 5/681; A61B 5/7425; A61B 5/743;**
A61B 5/6826; A61B 2503/40; A61B 2562/146

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 18.03.2021 US 202163162864 P

(62) Document number(s) of the earlier application(s) in accordance with Art. 76 EPC:
**22772329.3 / 4 307 994**

(71) Applicant: **Washington University
Saint Louis, MO 63130 (US)**

(72) Inventors:
• **O'BRIEN, Christine
St Louis (US)**

• **SHMUYLOVICH, Leonid
St Louis (US)**
• **ACHILEFU, Samuel
St Louis (US)**
• **BONETTA-MISTELI, Francesca
St Louis (US)**

(74) Representative: **Strange, Harry George
Withers & Rogers LLP
2 London Bridge
London, Greater London SE1 9RA (GB)**

Remarks:
This application was filed on 11.09.2025 as a divisional application to the application mentioned under INID code 62.

(54) **HEMODILUTION DETECTOR**

(57) Systems, devices, and methods for monitoring for postpartum hemorrhage (PPH) in a subject are disclosed. The disclosed system includes a laser speckle flow index sensor to monitor peripheral perfusion of the subject, and a multispectral Hb sensor to monitor intravascular hemoglobin concentration of the subject. The LSFI (laser speckle flow index) sensor uses laser speckle imaging of peripheral skin and muscle tissues to monitor peripheral perfusion.

**FIG. 1A**

EP 4 640 142 A2

# EP 4 640 142 A2

**Description**

CROSS-REFERENCE TO RELATED APPLICATIONS

This application claims priority to US provisional application number 63/162,864 filed on March 18, 2021, the content of which is incorporated by reference in its entirety.

STATEMENT REGARDING FEDERALLY SPONSORED RESEARCH OR DEVELOPMENT

This invention was made with government support under CA171651 awarded by the National Institutes of Health. The government has certain rights in the invention.

MATERIAL INCORPORATED-BY-REFERENCE

Not applicable.

FIELD OF THE DISCLOSURE

The present disclosure generally relates to systems, devices, and methods for non-invasively monitoring blood-related early indicators in a subject.

BACKGROUND OF THE DISCLOSURE

Postpartum hemorrhage (PPH), defined as the loss of 1 L of blood or more within 24 hours after birth, is the leading cause of maternal mortality worldwide with an estimated 14 million cases each year resulting in 130,000 deaths. Importantly, PPH has been noted as the most preventable cause of maternal mortality. The leading factors causing preventable PPH are delays in diagnosis and treatment. PPH prevention is especially critical in low resource settings that often have low blood stores for transfusion and consequently rely primarily on early pharmacologic treatment for PPH. The United States (US) has the highest maternal mortality rate of any developed country, where the most commonly used method for PPH diagnosis is a visual estimation of blood loss, a method known to underestimate blood loss. There is an urgent need for an early and accurate PPH alert system.

Other objects and features will be in part apparent and in part pointed out hereinafter.

DESCRIPTION OF THE DRAWINGS

Figure 1A is a schematic diagram illustrating the acquisition of a laser speckle flow index (LSFI) image from a human finger.

Figure 1B contains laser speckle flow index (LSFI) images of a middle and ring finger with the left side (left) or right side (right) wrapped with a band.

Figure 2A is a spectroscopic image measuring absorption between 880 - 1700 nm of swine whole blood diluted by 10% and 20% with PBS control.

Figure 2B is the ratio of 1020 to 1350 nm absorption in whole and diluted swine blood along with PBS control, which revealed significant differences between groups.

Figure 3 is a schematic illustration of the measurement of a laser speckle flow index from a swine wrist using source-detector offset to monitor skin and muscle perfusion during hemorrhage.

Figure 4 is a photo of a wearable laser speckle sensor in one aspect placed on a swine wrist and used during swine hemorrhage experiment.

Figure 5A shows short wave infrared spectra of varying concentrations of hemoglobin in saline.

Figure 5B is the ratio of 1020:1350 nm vs. hemoglobin concentration taken from the spectra in Figure 5A.

Figure 6 contains CAD renderings and pictures of a wearable hemodilution sensor in one aspect.

Figure 7A is a graph showing swine hemorrhage blood loss before and after a crystalloid infusion protocol. Between 90 and 115 minutes the laser speckle sensor was misaligned resulting in spurious signal (gray).

Figure 7B is a graph showing Laser speckle flow index (LSFI) results from the swine hemorrhage study illustrated in Figure 7A showing a correlation with blood withdrawal and crystalloid infusion. Between 90 and 115 minutes the laser speckle sensor was misaligned resulting in spurious signal (gray).

Figure 7C is a graph showing the normalized LSFI results throughout the hemorrhage study of Figure 7A. Between 90 and 115 minutes the laser speckle sensor was misaligned resulting in spurious signal (gray).

Figure 8 contains a series of graphs showing LSFI vs time, calculated at 10 and 100 frames per second, using either a ones square matrix convolution (top row) or an identity matrix convolution (bottom row), and using full image size, half image size, or quarter image size cropping (encompassing 12 distinct convolutions).

Figure 9A is a graph of swine hemorrhage blood loss before and after a crystalloid infusion protocol.

Figure 9B is a graph showing swine hemorrhage blood loss before and after a crystalloid infusion protocol.

Figure 9C is a graph showing an unsmoothed laser speckle flow index (LFSI) resulting from the swine hemorrhage study of Figure 9A, showing a correlation with blood withdrawal and crystalloid infusion.

Figure 9D is a graph showing a smoothed laser speckle flow index (LSFI) result from the swine hemorrhage study in Figure 9C, showing a correlation with blood withdrawal and crystalloid infusion.

Figure 9E is a graph showing pre-vein collapse blood loss vs. LSFI from unsmoothed LSFI data.

Figure 9F is a graph showing pre-vein collapse blood loss vs. LSFI from smoothed LSFI data.

Figure 9G is a graph showing post-vein collapse blood loss vs. LSFI from unsmoothed LSFI data.

Figure 9H is a graph showing post-vein collapse blood loss vs. LSFI from smoothed LSFI data.

Figure 9I is a graph showing crystalloid infusion vs. LSFI from unsmoothed LSFI data.

Figure 9J is a graph showing crystalloid infusion vs. LSFI from smoothed LSFI data.

Figure 10 contains a series of graphs showing systolic blood pressure (SBP), diastolic blood pressure (DBP), pulse pressure (SBP-DBP), temperature, heart rate (HR), and respiratory rate over time during a swine hemorrhage study (top row), corresponding normalized vital signs (middle row), and blood volume (bottom row). With the exception of temperature, vital signs parameters demonstrated a decrease with blood loss (blue), and an increase with crystalloid administration (red).

Figure 11 contains a series of graphs that correlate normalized vital signs and blood loss. Top panel includes all time points, and the bottom panel includes times preceding vein collapse.

Figure 12 is a correlation heat map for LSFI and vital signs during swine blood loss.

Figure 13 is a graph showing speckle plethysmography (SPG) and photoplethysmography (PPG) traces from a swine hemorrhage study. The SPG signal has higher SNR compared to PPG, although both waveforms are data rich. The time delay ($\Delta t$) between peaks from SPG and PPG has been shown to correspond with systemic vascular resistance, and can be easily calculated using the disclosed wearable laser speckle sensor.

Figure 14 contains CAD renderings of a completely wearable laser speckle sensor in one aspect.

Those of skill in the art will understand that the drawings, described below, are for illustrative purposes only. The drawings are not intended to limit the scope of the present teachings in any way.

There are shown in the drawings arrangements that are presently discussed, it being understood, however, that the present embodiments are not limited to the precise arrangements and are instrumentalities shown. While multiple embodiments are disclosed, still other embodiments of the present disclosure will become apparent to those skilled in the art from the following detailed description, which shows and describes illustrative aspects of the disclosure. As will be realized, the invention is capable of modifications in various aspects, all without departing from the spirit and scope of the present disclosure. Accordingly, the drawings and detailed description are to be regarded as illustrative in nature and not restrictive.

DETAILED DESCRIPTION

In various aspects, systems, devices, and methods for monitoring a subject for hemorrhage including, but not limited to post-partum hemorrhage (PPH), are disclosed herein.

During hemorrhage, two important compensatory mechanisms occur: 1) blood is shunted from the periphery to vital organs by constricting peripheral vessels; 2) interstitial fluid is transferred into vessels to maintain blood volume, effectively reducing hemoglobin (Hb) concentration and hematocrit (Hct). These compensatory mechanisms help stabilize the patient and delay the time until global vascular indicators such as blood pressure and heart rate are affected. Thus, the monitoring of peripheral blood flow and blood content can detect relatively minor decreases in Hb and Hct that serve as early indicators of hemorrhage.

Optical technologies are well suited to noninvasively measure blood flow and blood content. Laser speckle imaging directly measures flowing blood cells and the laser speckle flow index (LSFI) is proportional to velocity. Optical spectroscopy provides quantification of blood and tissue oxygenation (near-infrared region), as well as quantification of water (infrared region), enabling observation of water transfer to the vasculature during hemorrhage. Optical monitoring techniques are non-ionizing, label-free, fast, and can be implemented using small and wearable devices to provide a continuous ergonomic sensing system. Preliminary experiments described in the Examples below demonstrate sensitivity to reduced perfusion in vivo using laser speckle imaging, and optical spectroscopy measures significant differences between blood samples diluted with saline to physiologic levels seen in PPH.

Optical monitoring of blood flow and blood content has numerous advantages: sensitivity to multiple intrinsic biological chromophores (melanin, deoxy- and oxyhemoglobin, lipids, proteins, and water) depending upon the optical wavelengths used; ability to detect and quantify blood flow; high potential for small, simple, and wearable hardware; and rapid results. Such characteristics are ideal for patient monitoring, as evidenced by the pulse oximeter, an optical device used globally for patient monitoring. Optical spectroscopy-based tools have been developed for in vitro and in vivo measurement of hemoglobin, and continuous noninvasive optical spectroscopy tools have been used extensively in critical care patients to monitor changes in Hb concentration caused by hypovolemia. Although the perfusion index is known to be skewed and has high patient variability, previous results are encouraging and show that non-invasive optical measures can detect early signs of postpartum blood loss.

In various aspects, a multifunctional sensing system to track Hb concentration and peripheral perfusion for the monitoring and/or early detection of postpartum hemorrhage (PPH) is disclosed. The disclosed system includes an LSFI (laser speckle flow index) sensor and a multispectral Hb sensor. In various aspects, the disclosed system synergistically combines laser speckle imaging for blood perfusion measurements and NIR/SWIR spectroscopy for monitoring Hb, to provide tracking of the two separate and independent compensatory mechanisms of PPH.

The LSFI (laser speckle flow index) sensor uses laser speckle imaging of peripheral skin and muscle tissues to monitor peripheral perfusion. The laser speckle sensor performs peripheral perfusion monitoring which is proportional to blood flow velocity to provide a more direct measure of perfusion than the perfusion index. In one aspect, the LSFI sensor includes a 785 nm laser diode and a video camera to obtain laser speckle contrast images. The laser speckle contrast images are processed using established algorithms to obtain laser speckle flow index images indicative of peripheral perfusion. In some aspects, the LSFI sensor is a wearable LSFI (laser speckle flow index) sensor positioned over a muscle and gently held in place with a band, ensuring placement over muscles in order to measure both skin and muscle blood flow.

The disclosed multispectral Hb sensor of the disclosed system uses near-infrared (NIR) absorption of Hb and short-wave infrared (SWIR) absorption of water; absorption by water in the SWIR range is stronger than within the spectral ranges used by existing devices, thereby improving sensitivity and specificity of water measurements obtained using the disclosed system. In some aspects, the multispectral Hb sensor of the disclosed system includes two light-emitting diodes (LEDs) at different wavelengths that are used to monitor blood content. In one aspect, an 800 nm LED (L1) is used for Hb measurement, as this is the point where oxy- and deoxyhemoglobin absorb at the same rate, eliminating variability caused by the oxygen saturation of Hb to focus solely on total hemoglobin. For water measurements, a 1340 nm LED (L2) is used to balance between high contrast and subcutaneous penetration depth. The multispectral Hb sensor further includes two photodiodes for detecting LED light: one sensitive to NIR Hb signal (D1, silicon detector), and one sensitive to SWIR water signal (D2, InGaAs detector). Ratiometric calculations and computational removal of room light contamination are performed using algorithms similar to those used in pulse oximeters. In some aspects, the multispectral Hb sensor may further include a microprocessor to perform de-noising and ratiometric calculations throughout data capture. For pulsatile blood flow, ratiometric calculations and removal of room lighting are performed using algorithms similar to those used in standard pulse oximetry to extract the Hb to water ratio.

In various aspects, data obtained using the sensors of the disclosed multispectral Hb sensor are transferred to secure cloud storage using Bluetooth Low Energy (BLE) wireless network.

Additional description of the disclosed system, devices, and methods are provided in the Examples below.

**Computing Systems and Devices**

As will be appreciated based upon the foregoing specification, the above-described aspects of the disclosure may be implemented using computer programming or engineering techniques including computer software, firmware, hardware or any combination or subset thereof. Any such resulting program, having computer-readable code means, may be embodied or provided within one or more computer-readable media, thereby making a computer program product, i.e., an article of manufacture, according to the discussed aspects of the disclosure. The computer-readable media may be, for example, but is not limited to, a fixed (hard) drive, diskette, optical disk, magnetic tape, semiconductor memory such as read-only memory (ROM), and/or any transmitting/receiving medium, such as the Internet or other communication network or link. The article of manufacture containing the computer code may be made and/or used by executing the code directly from one medium, by copying the code from one medium to another medium, or by transmitting the code over a network.

These computer programs (also known as programs, software, software applications, "apps", or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The "machine-readable medium" and "computer-readable medium," however, do not include transitory signals. The term "machine-readable signal" refers to any signal used to provide machine instructions and/or data to a programmable processor.

As used herein, a processor may include any programmable system including systems using micro-controllers, reduced instruction set circuits (RISC), application specific integrated circuits (ASICs), logic circuits, and any other circuit or processor capable of executing the functions described herein. The above examples are examples only, and are thus not intended to limit in any way the definition and/or meaning of the term "processor."

As used herein, the terms "software" and "firmware" are interchangeable, and include any computer program stored in memory for execution by a processor, including RAM memory, ROM memory, EPROM memory, EEPROM memory, and non-volatile RAM (NVRAM) memory. The above memory types are example only, and are thus not limiting as to the types of memory usable for storage of a computer program.

In one aspect, a computer program is provided, and the program is embodied on a computer-readable medium. In one aspect, the system is executed on a single computer system, without requiring a connection to a server computer. In a further aspect, the system is being run in a Windows® environment (Windows is a registered trademark of Microsoft Corporation, Redmond, Washington). In yet another aspect, the system is run on a mainframe environment and a UNIX® server environment (UNIX is a registered trademark of X/Open Company Limited located in Reading, Berkshire, United Kingdom). The application is flexible and designed to run in various different environments without compromising any major functionality.

In some aspects, the system includes multiple components distributed among a plurality of computing devices. One or more components may be in the form of computer-executable instructions embodied in a computer-readable medium. The systems and processes are not limited to the specific aspects described herein. In addition, components of each system and each process can be practiced independent and separate from other components and processes described herein. Each component and process can also be used in combination with other assembly packages and processes. The present aspects may enhance the functionality and functioning of computers and/or computer systems.

Definitions and methods described herein are provided to better define the present disclosure and to guide those of ordinary skill in the art in the practice of the present disclosure. Unless otherwise noted, terms are to be understood according to conventional usage by those of ordinary skill in the relevant art.

In some embodiments, numbers expressing quantities of ingredients, properties such as molecular weight, reaction conditions, and so forth, used to describe and claim certain embodiments of the present disclosure are to be understood as being modified in some instances by the term "about." In some embodiments, the term "about" is used to indicate that a value includes the standard deviation of the mean for the device or method being employed to determine the value. In some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable. The numerical values presented in some embodiments of the present disclosure may contain certain errors necessarily resulting from the standard deviation found in their respective testing measurements. The recitation of ranges of values herein is merely intended to serve as a shorthand method of referring individually to each separate value falling within the range. Unless otherwise indicated herein, each individual value is incorporated into the specification as if it were individually recited herein. The recitation of discrete values is understood to include ranges between each value.

In some embodiments, the terms "a" and "an" and "the" and similar references used in the context of describing a particular embodiment (especially in the context of certain of the following claims) can be construed to cover both the singular and the plural, unless specifically noted otherwise. In some embodiments, the term "or" as used herein, including the claims, is used to mean "and/or" unless explicitly indicated to refer to alternatives only or the alternatives are mutually exclusive.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and can also cover other unlisted steps. Similarly, any composition or device that "comprises," "has" or "includes" one or more features is not limited to possessing only those one or more features and can cover other unlisted features.

All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the present disclosure and does not pose a limitation on the scope of the present disclosure otherwise claimed. No language in the specification should be construed as indicating any non-claimed element essential to the practice of the present disclosure.

Groupings of alternative elements or embodiments of the present disclosure disclosed herein are not to be construed as limitations. Each group member can be referred to and claimed individually or in any combination with other members of the group or other elements found herein. One or more members of a group can be included in, or deleted from, a group for reasons of convenience or patentability. When any such inclusion or deletion occurs, the specification is herein deemed to contain the group as modified thus fulfilling the written description of all Markush groups used in the appended claims.

Any publications, patents, patent applications, and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present disclosure.

Having described the present disclosure in detail, it will be apparent that modifications, variations, and equivalent embodiments are possible without departing the scope of the present disclosure defined in the appended claims. Furthermore, it should be appreciated that all examples in the present disclosure are provided as non-limiting examples.

## EXAMPLES

The following example illustrates various aspects of the disclosure. *Example 1: Development and Validation of a Post-Partum Hemorrhage Monitor*

To develop and validate a post-partem (PPH) hemorrhage monitoring system, the following experiments were conducted..

Laser speckle imaging

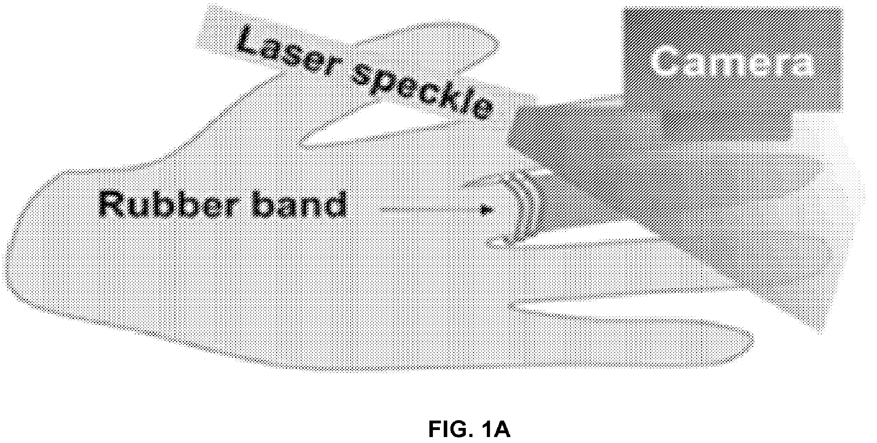

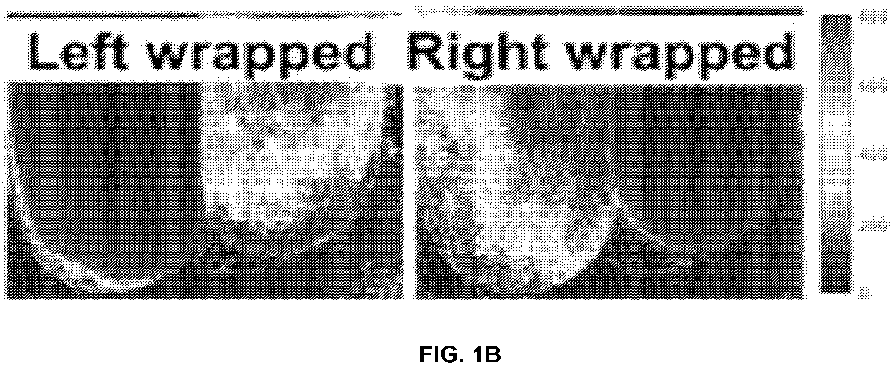

A laser speckle imaging system using a 785 nm laser diode and video camera was assembled as illustrated in Figure 1A. To test the ability of the system to measure reduced blood flow, the middle finger of a volunteer was wrapped tightly with a rubber band to disrupt blood flow and then the wrapped middle finger and the adjacent unwrapped ring finger were measured using the camera (Fig. 1A). This experiment was repeated with the ring finger wrapped and the middle finger unwrapped. Laser speckle contrast images were processed using established algorithms and the resulting laser speckle flow index images are shown in Fig. 1B.

SWIR spectroscopy

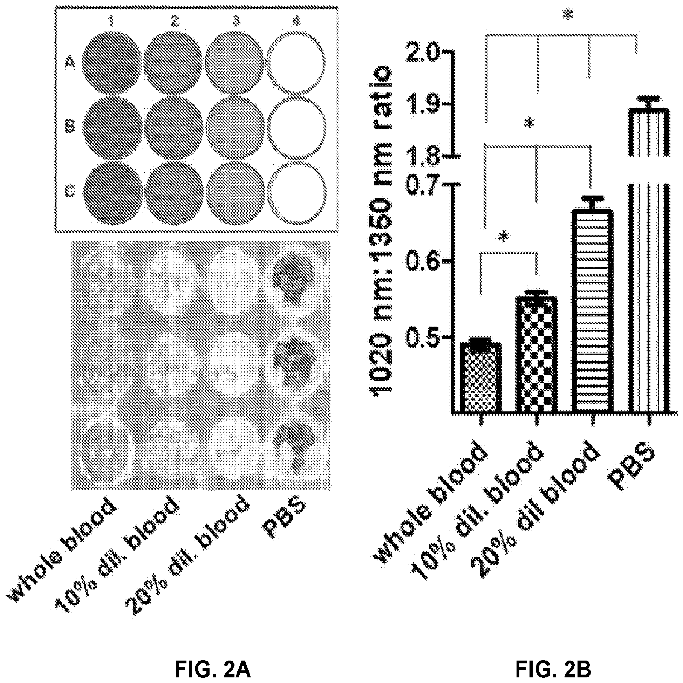

The ability of SWIR spectroscopy using a hyperspectral imaging system to distinguish varying levels of Hb was tested using swine blood diluted with PBS. Wells of whole blood, 10% diluted blood, 20% diluted blood, and PBS were measured in triplicate (Figure 2A). T hyperspectral imaging system was used to image absorption at wavelengths spanning 880-1700 nm. The ratio of the image at 1020 nm and 1350 nm was calculated and is shown in Fig. 2A. The intensity ratios in each well were quantified and the mean and standard deviation of the triplicate measures are summarized in Fig. 2B. Statistically significant increases were observed across every group as measured by ANOVA with Bonferroni's multiple comparison test, demonstrating efficacy of this technique for quantifying differences in Hb concentration as measured via water to Hb ratio (1020 nm : 1330 nm).

Wearable laser speckle sensor design V1 and components

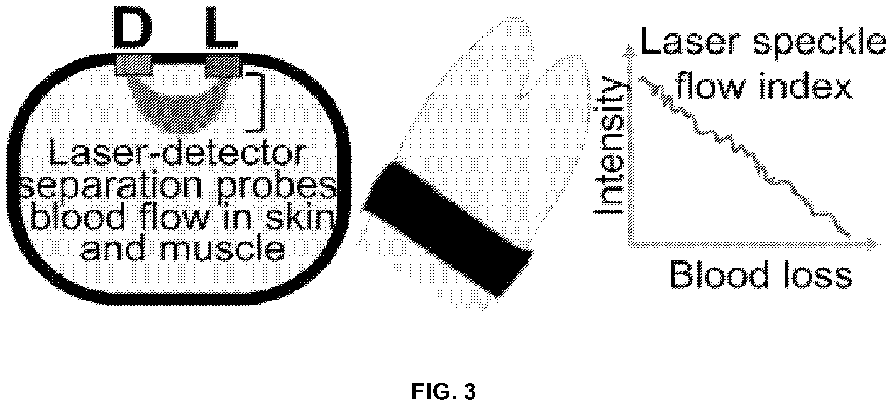

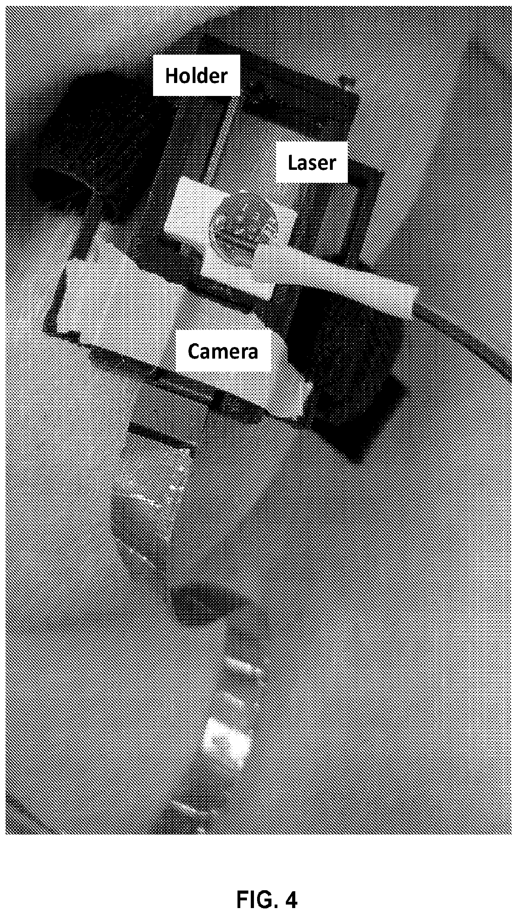

The laser speckle sensor is an optical sensor designed in reflectance mode, i.e. the light source and detector are positioned on the same side, however a transmission mode design could also be used. The reflection mode device, illustrated in FIG. 4, includes of a 50 mW 780 nm laser module (Laserland, 11071013) and a double-lens Raspberry Pi camera sensor. The double lens camera sensor consists of a Raspberry Pi Camera Module V2, with the lens turned to have a maximal focal distance, and a second lens, from a second V2 module, inverted and attached directly to the surface of the first lens. A 0.4 mm sapphire window (Edmund Optics, 43-628) is attached to the second lens, allowing for the sensor to be in focus on objects sitting or pressing directly on the window. The laser and double-lens camera sensor are held in position to be directly in contact with a subject by a 3D printed holder. The 3D printed holder is adjustable and allows for the laser and sensor distance to be varied to a specified distance and then fixed, for optimization of signal intensity and contrast. The laser module is powered by a 3.3 V wall source, but can be powered by a battery. The camera sensor is powered and controlled by a Raspberry Pi 4 Model 4 computer board. A schematic and photo of this device is shown in Figures 3 and 4, respectively.

Hemodilution sensor design and components

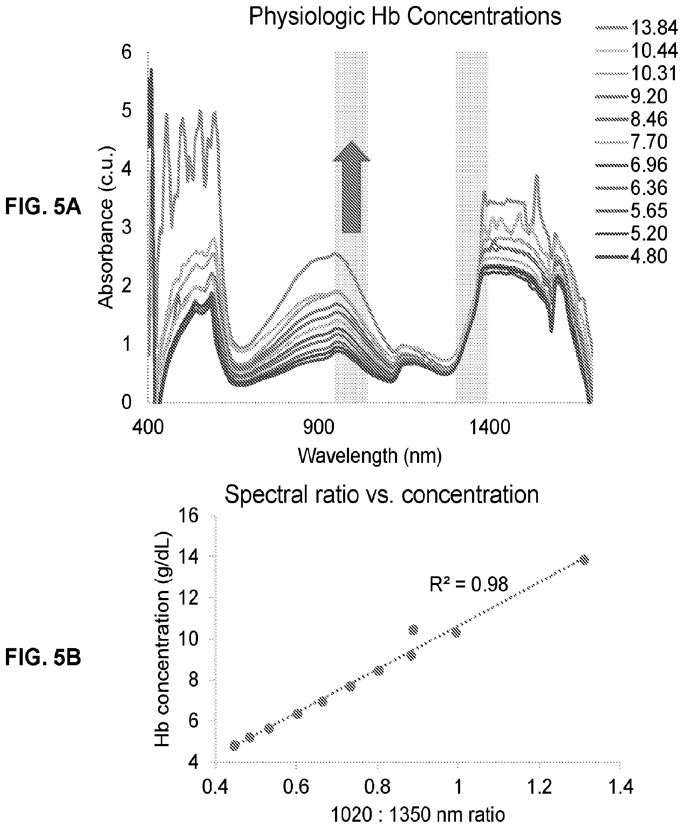

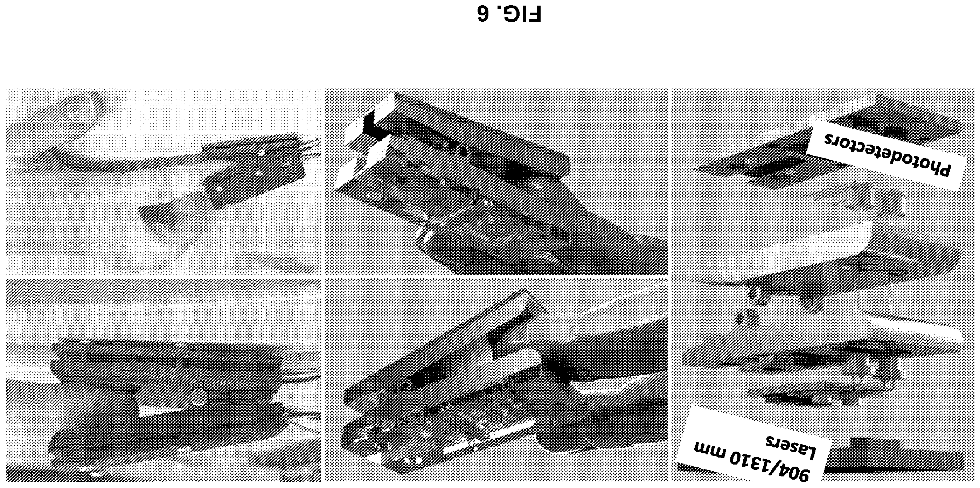

The wavelengths chosen for use in the hemodilution sensor were based on preliminary data using a short wave infrared spectrometer that measured spectral changes across hemoglobin samples ranging in concentration from 4.8 - 13.84 g/dL (Figure 5A). The results revealed significant increases across 850 - 1000 (attributed to hemoglobin) and a constant response between 1300 - 1350 (attributed to water) across the concentrations. The ratio of intensities at two wavelengths in these bands, 1020nm and 1350 nm, were plotted against hemoglobin concentration (Figure 5B) and an $R^2$=0.98 was achieved; lasers from these bands were chosen for use in the wearable hemodilution sensor design. The hemodilution sensor (Figure 6) consists of two InGaAs photodiode detectors (although could be completed with one) with sensitivity from 800-1700 nm (Thorlabs, FGA01), directly across from a 904nm laser (Thorlabs, L904P010 ) and a 1310 nm laser (Thorlabs, ML725B8F) which were each connected to a laser driver to maintain constant current and therefore constant optical output (IC Haus, WK2D) (Figure 6). The two light sources were modulated at 25 Hz in alternating 20 ms intervals using digital pins from an Arduino Uno, and chosen to detect relative changes in hemoglobin and water. The photodiode detected the transmitted optical signals via an analog pin on the Arduino Uno, which has a 10 bit ADC. Arduino code controlled the light source modulation and separated the 900 nm and 1310 nm signals into distinct channels and plotted their output in real time. The Arduino was plugged into a laptop computer via USB/USB B connection. The photodiode bias voltage was supplied using an external wall mounted power supply of 12 V, but could be powered by a battery. Although the current design is in transmission mode, it could be built in reflectance mode.

Swine hemorrhage protocol

A twelve week old male White Yorkshire x Landrace pig was anesthetized and cut downs were performed on the femoral vein and artery to establish a blood withdrawal port and to insert an arterial blood pressure catheter, respectively. The estimated blood volume (EBV, 58-74 mL/kg) was calculated (2100-2500 mL) based on the swine weight (34.5 kg). Once the catheter was in place, the hemodilution sensor (Figure 6) was placed on the ear of the pig and the laser speckle sensor was placed on the left posterior hock after removing hair with an electrical hair trimmer (Figure 3). The sensors recorded baseline levels for 15 minutes, after which we removed 1.5% blood volume (33 mL) every 5 minutes, followed by a 2 mL saline flush. This continued for a total of 800 mL blood removed over the course of 2.5 hours, which was an estimated blood loss of ~31-38%. In parallel, heart rate, systolic and diastolic blood pressure, temperature, blood oxygen saturation, respiratory rate and hematocrit were measured every 15 minutes throughout the procedure. After reaching 800 mL of blood loss, the swine was then reinfused every 5 minutes with 33 mL of crystalloids for a total of 264 mL over 35 minutes. Upon completion of the crystalloid infusion, the swine was euthanized using intravenous potassium chloride overdose.

Swine hemorrhage laser speckle imaging methods

Data was collected using a Python script from the Raspberry Pi. Data was collected via video for 10 seconds every minute from 15 minutes before the start of the blood loss protocol, until 5 minutes after the final crystalloid infusion. Video data was saved directly onto the Raspberry Pi hard drive. Data was processed post-study as a rolling average of the speckle index over time. The camera was set to capture video at 100 frames per second, with a 5 ms exposure time.

Laser speckle flow index algorithm

In laser speckle contrast imaging, contrast is generated by applying a spatial averaging algorithm within a square sliding window that spans a raw speckle image. Specifically, to find the speckle contrast at a given pixel $(x,y)$, one defines a square window centered about $(x,y)$ and divides the standard deviation of the pixel intensity within that window by the mean pixel intensity within the window. For real time processing of video speckle data, this algorithm must be applied to every video frame, and with frame rates up to 100 fps, efficient speckle contrast algorithms are critical. The standard deviation of pixel intensity within each sliding window is related to the variance of the pixel intensity, which is determined by taking the difference between the mean of the square of the raw image pixel intensity and square of the mean of the raw image pixel intensity. An established approach for efficiently determining these rolling averaged images is convolving a square array of ones with both the square of the raw image and with the raw image itself, resulting in the following expression for the speckle contrast image pixel intensity (k):

$$k = \frac{\sqrt{\dfrac{n^2 I_S^2 \otimes \begin{pmatrix} 1 & \cdots & 1 \\ \vdots & 1 & \vdots \\ 1 & \cdots & 1 \end{pmatrix} - \left( I_S \otimes \begin{pmatrix} 1 & \cdots & 1 \\ \vdots & 1 & \vdots \\ 1 & \cdots & 1 \end{pmatrix} \right)^2}{n^2(n^2-1)}}}{\dfrac{1}{n^2} I_S \otimes \begin{pmatrix} 1 & \cdots & 1 \\ \vdots & 1 & \vdots \\ 1 & \cdots & 1 \end{pmatrix}} \quad \text{(Equation 1)}$$

where the sliding windows have dimensions $n \times n$ ($n$ is odd without loss of generality), $I_s$ is the raw image intensity, and the ones matrices have the same dimension as the sliding window. The disclosed hemorrhage monitoring system captures a video stream, where each frame is a raw intensity image. To detect peripheral vascular flow, each frame is analyzed according to Equation 1 to yield a speckle contrast image $k$, and then the average pixel intensity $<k>$ across the entire image (excluding a (n-1)/2 thick rectangular border) is calculated and stored for each frame. Thus, the output signal is a single averaged speckle contrast value over time.

For each captured frame, the average speckle contrast $<k>$ can be implemented in python using methods from publicly available software libraries like *Numpy.mean(), Numpy.ones() Scipy.signal.convolve2d(),* or *Scipy.signal.fftconvolve*(). However, because the relevant output signal for monitoring is a measure of average speckle contrast and not the speckle contrast image itself, an alternative approach is possible that significantly speeds up processing time. Consider an alternative speckle contrast index $k'$:

$$k' = \frac{\sqrt{\dfrac{n I_S^2 \otimes \begin{pmatrix} 1 & \cdots & 0 \\ \vdots & 1 & \vdots \\ 0 & \cdots & 1 \end{pmatrix} - \left( I_S \otimes \begin{pmatrix} 1 & \cdots & 0 \\ \vdots & 1 & \vdots \\ 0 & \cdots & 1 \end{pmatrix} \right)^2}{n(n-1)}}}{\dfrac{1}{n} I_S \otimes \begin{pmatrix} 1 & \cdots & 0 \\ \vdots & 1 & \vdots \\ 0 & \cdots & 1 \end{pmatrix}} \quad \text{(Equation 2)}$$

where the ones matrices in Equation 1 are replaced with $n \times n$ identity matrices ($n$ is odd). This speckle contrast index $k'$ is based on averages of the raw speckle image intensity and square of the raw image intensity along the diagonal of the square window (effectively replacing each pixel $(x,y)$ with the sum of $n$ pixels along a diagonal with $(x,y)$ at the center):

$$I_{S(x,y)} = \sum_{-\frac{n-1}{2}}^{\frac{n-1}{2}} I_{S(x+k,y+k)} \quad \text{(Equation 3)}$$

For a 7 by 7 square window, this diagonal average can be written directly for the raw image and square of the raw image, without applying convolutions, as:

$$\langle I_S \rangle' = (I_S[6{:}h-1,6{:}w-1] + I_S[5{:}h-2,5{:}w-2] + I_S[4{:}h-3,4{:}w-3] +$$
$$I_S[3{:}h-4,3{:}w-4] + I_S[2{:}h-5,2{:}w-5] + I_S[1{:}h-6,1{:}w-6] + I_S[0{:}h-7,0{:}w-7]) \qquad \text{(Equation 4)}$$

$$\langle I_S^2 \rangle' = (I_S^2[6{:}h-1,6{:}w-1] + I_S^2[5{:}h-2,5{:}w-2] + I_S^2[4{:}h-3,4{:}w-3] +$$
$$I_S^2[3{:}h-4,3{:}w-4] + I_S^2[2{:}h-5,2{:}w-5] + I_S^2[1{:}h-6,1{:}w-6] +$$
$$I_S^2[0{:}h-7,0{:}w-7]) \qquad \text{(Equation 5)}$$

where the raw image $I_s$ dimensions are $h$ by $w$. Then Equation 2 can be rewritten:

$$k' = \frac{\sqrt{\frac{7*\langle I_S^2 \rangle' - (\langle I_S \rangle')^2}{7*6}}}{\frac{1}{7}\langle I_S \rangle'} \qquad \text{(Equation 6)}$$

In testing 100 fps video streams, Equation 6 was found to be 3-5 times faster than Equation 1. Furthermore this hard-coded approach has the added value of simplicity, without the need for 3rd party python libraries like Scipy for implementing efficient convolution.

Further improvements in processing speed can be realized by cropping the video stream data, for example from 640 x 480 to 320 x 240, 160 x 120 or smaller. In addition, the frame rate may be reduced to 30 fps, 10 fps, or smaller. Finally, the video stream that is captured can be captured in YUV mode rather than RGB mode. In YUV mode only the first third of the full frame bytes need to be utilized (the 'Y' channel) as this channel contains the pixel intensity, while the 'U' and 'V' channels contain pixel color. Using RGB video mode by contrast requires reading in 3 times more data per frame followed by conversion to gray scale for each captured frame.

Results

To monitor hemorrhage (and/or postpartum hemorrhage), a laser speckle flow index (LSFI) can be derived from the inverse square of the speckle contrast index:

$$LSFI = \frac{1}{\langle (k)^2 \rangle} \qquad \text{(Equation 7)}$$

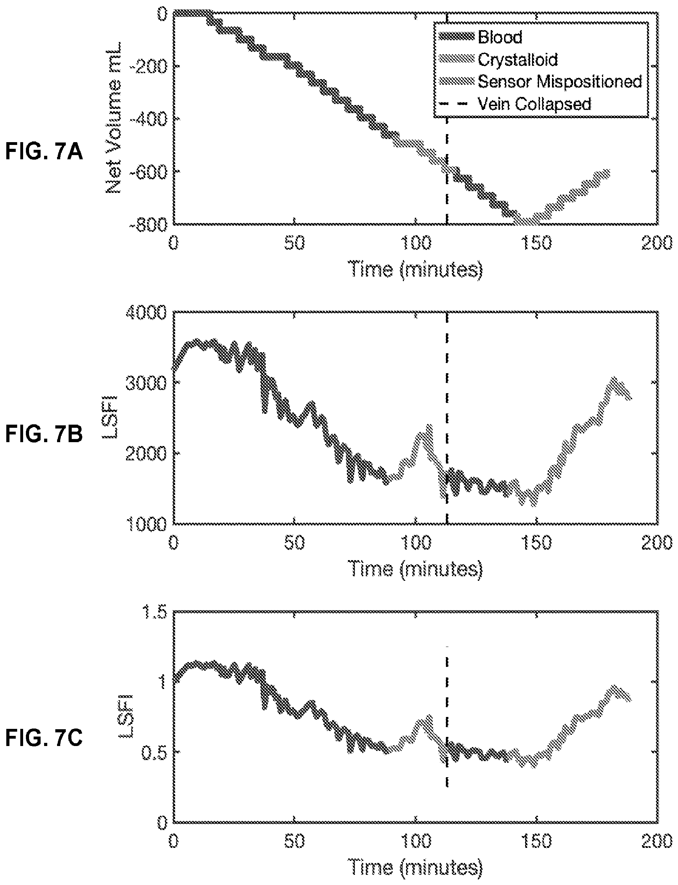

where $< k >$ is an averaged speckle contrast index. This average can be defined different ways. The average speckle contrast $<k>$ can be defined at a given time point as the average value across all pixels in a processed speckle video frame captured at that time point. One can also average across multiple frames, obtaining the average speckle contrast index over a 10 second time period, for example. We have tested the ability of LSFI to noninvasively detect hemorrhage-induced peripheral vasoconstriction due to physiologic compensatory mechanisms in a swine experimental model. After obtaining femoral vein access, nearly 800 mL of blood (~30% blood volume) was removed over a duration of 2 hours (33 mL blood every 5 minutes). Subsequently 231 mL of crystalloid was administered over a duration of 35 minutes (33 mL crystalloid every 5 minutes) (Figure 7). Of note, veins collapsed at 113 minutes from the start of the experiment (dotted vertical line).

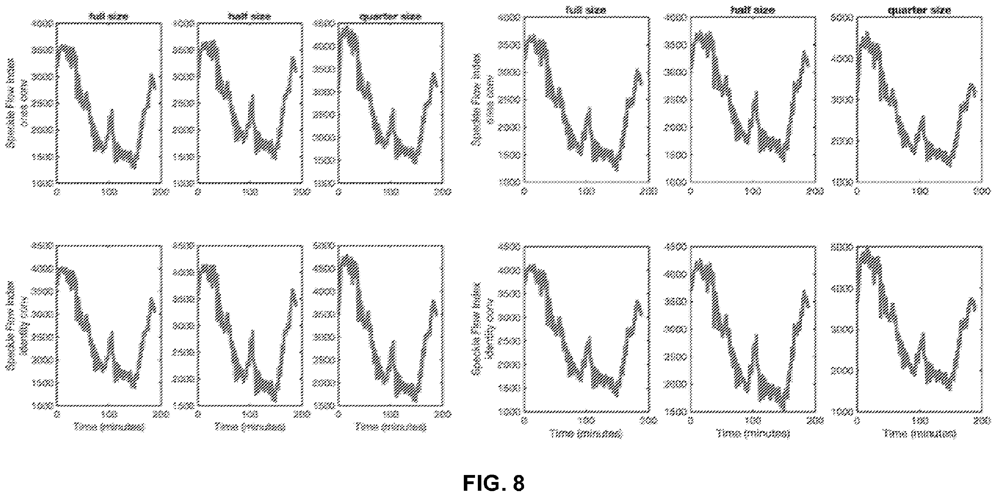

The hemorrhage monitor system was worn on the swine wrist, and 10 s of 320 x 240 100 fps speckle video was recorded every minute throughout the experiment. For each 10 s video, $<k>$ was calculated for each frame using the traditional speckle contrast algorithm based on convolution with a square ones matrix ($<k>$) (Equation 1), and the modified speckle contrast algorithm based on convolution with a square identity matrix ($<k'>$Identity))(*Equation* 6). In each calculation, the full 320 x 240 image size was used to define a full-image size $<k>_{FULL}$. In addition, a ½ cropped 160 x 120 image and ¼ cropped 80 x 60 image was used to extract half-image ($<k>_{HALF}$) and quarter-image ($<k>_{QUARTER}$) size speckle crop indexes, respectively. These full-size and cropped image approaches were applied to both $<k>$ and $<k'>$. Finally, the average value across all frames was calculated using the full 100fps video stream $<k>_{FPS\_100}$, as well as with a tenfold temporally down-sampled (effective 10fps) video stream $<k>_{FPS\_010}$. Taken together, there were 12 different $<k>$ values calculated for each 10s video stream based on type of convolution, type of image cropping, and type of effective frame rate used for data processing (Figure 8). These defined 12 different approaches to calculating LSFI values for each 10s video per Equation 7: $LSFI_{Ones,FULL,FPS\_100}$, $LSFI_{Ones,HALF,FPS\_100}$, $LSFI_{Ones,QUARTER,FPS\_100}$, $LSFI_{Ones,FULL,FPS\_010}$, $LSFI_{Ones,HALF,FPS\_010}$, $LSFI_{Identity,QUARTER,FPS\_010}$, $LSFI_{Identity,FULL,FPS\_100}$, $LSFI_{Identity,HALF,FPS\_100}$, $LSFI_{Identity,QUARTER,FPS\_100}$, $LSFI_{Identity,FULL,FPS\_010}$, $LSFI_{Identity,HALF,FPS\_010}$, and $LSFI_{Identity,QUARTER,FPS\_010}$.

The noninvasively derived LSFI signal maintained a steady baseline for 15 minutes prior to blood draw, decreased with decreasing blood volume, and increased with addition of crystalloid (Figure 7). Between 90 and 115 minutes the speckle sensor was misaligned, resulting in an erroneous LSFI signal (gray portion in Figure 7). The shape of the LSFI signal over time remained consistent regardless of type of convolution, degree of image cropping, or effective frame rate (Figure 8), and a cross correlation of the 12 variations of LSFI against each other yielded a correlation coefficient R of 0.99 -1.00, indicating that significant reductions in processing burden can be achieved (by decreasing frame rate, analyzing a cropped image, and averaging across a diagonal square matrix rather than a full square matrix), without compromising LSFI signal integrity.

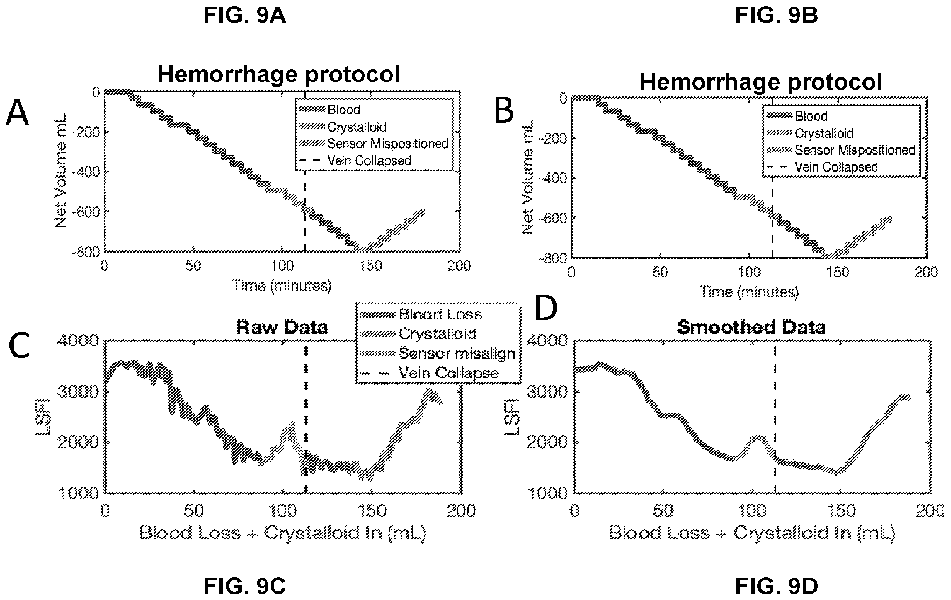

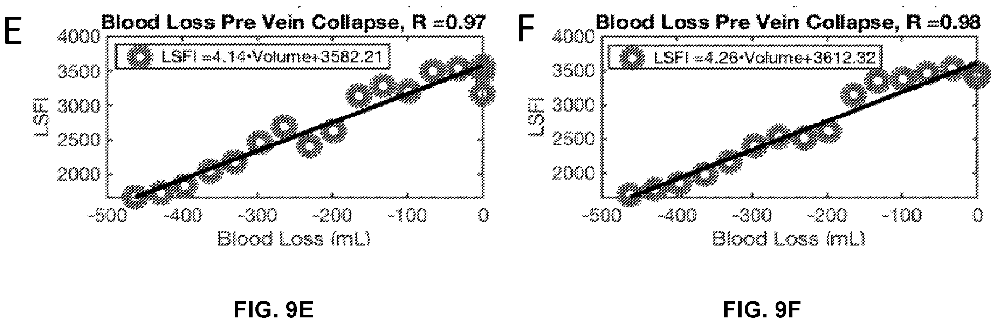

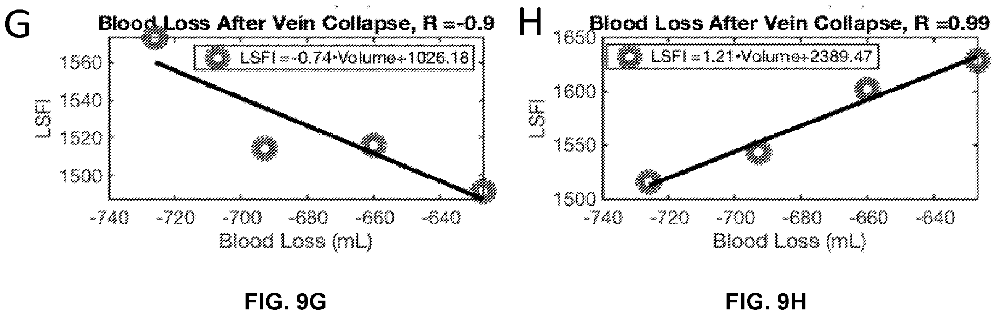

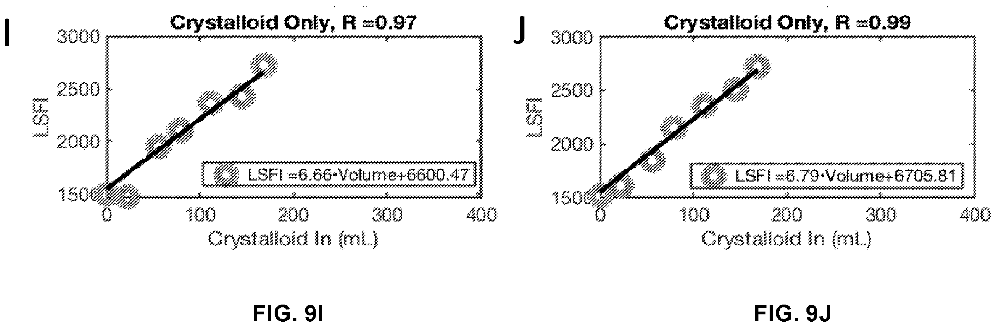

A linear regression between unsmoothed and smoothed (10 point moving average) LSFI signal and net fluid volume was determined for each LSFI variation (Figure 9). LSFI showed a strong linear correlation with fluid volume change during the entire experiment (Pearson R=0.88). However, the regression between LSFI and fluid volume change during the first 2 hours, where blood volume decreased, was different from the regression between LSFI and fluid volume change in the last 35 minutes, where blood volume was fixed and crystalloid was added. Specifically, the regression between $LSFI_{Ones,FULL,FPS\_100}$ and blood loss volume prior to vein collapse showed an R=0.97 in unsmoothed data and R=0.98 in smoothed data, with a slope of 4.14/mL and 4.24/mL, respectively (Figures 9E and 9F). During blood loss post-vein collapse, the regression coefficient between $LSFI_{Ones,FULL,FPS\_100}$ and blood loss was R=-0.9 in unsmoothed data and R=0.99 in smoothed data, with a slope of -0.74/mL and 1.21/mL, respectively (Figures 9G and 9H). Meanwhile, the regression between $LSFI_{Ones,FULL,FPS\_100}$ and crystalloid input volume was R=.97 in unsmoothed data and R=0.99 in smoothed data, with a slope of 6.66/mL and 6.79/mL, respectively (Figures 9I and 9J). Similar strong but distinct correlations for LSFI vs blood loss volume and LSFI vs crystalloid input volume were found with the 11 other variations of LSFI processing.

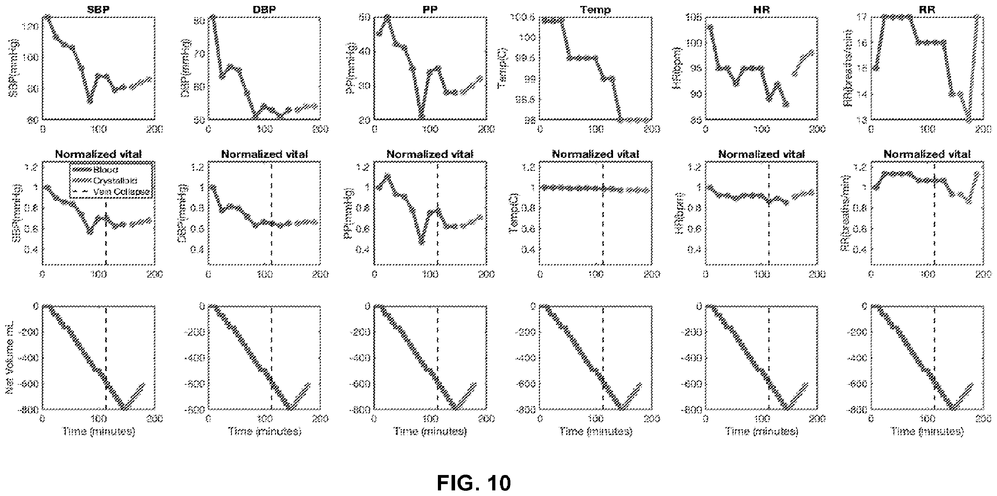

Vital signs, including systolic and diastolic blood pressure, pulse pressure, temperature, heart rate, and respiratory rate were recorded noninvasively at 15 minute intervals, and appeared to show a similar downward trend with blood loss followed by upward trend with administration of crystalloid (Figure 10). Vital signs were obtained at only 3 time points during crystalloid infusion, making linear correlation between vital signs and crystalloid volume challenging to interpret. The middle panel shows normalized vital signs, where each value was divided by the initial value.

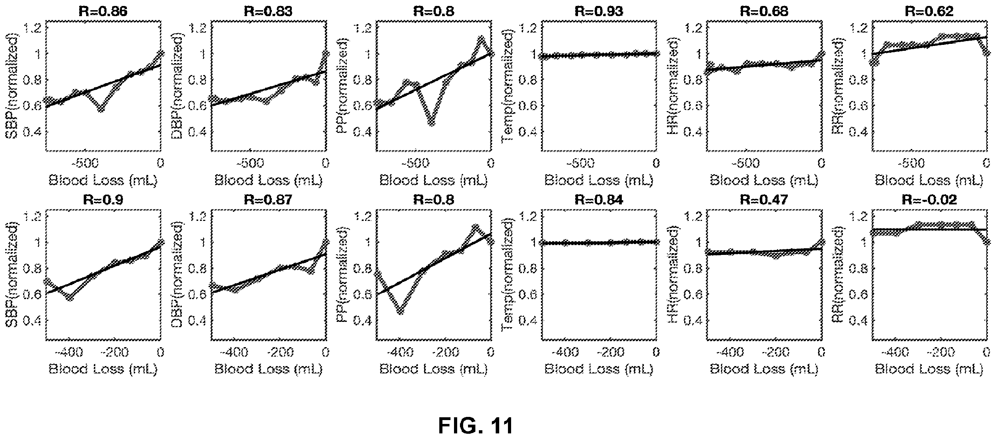

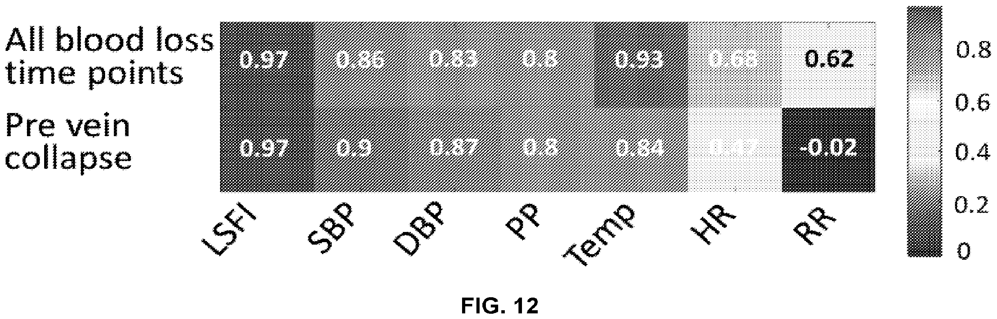

Figure 11 demonstrates the correlation between volume of blood loss and each measured normalized vital sign, with the top panel including all time points and the bottom panel including times preceding vein collapse and before crystalloid infusion. While body temperature showed the strongest correlation with blood volume loss, unlike LSFI, body temperature showed no increase with crystalloid infusion (Figure 11). Indeed body temperature may have decreased steadily with time as a result of anesthesia rather than as a result of blood loss. Future studies will include control swine that are anesthetized for the same duration as the hemorrhage protocol to track changes caused by anesthesia over time. LSFI had the highest correlation to blood loss (R=0.97) and crystalloid infusion (R=0.97) of all the vital signs tracked (Figure 12), underscoring the accuracy and added value of this technique to monitor dynamic changes in peripheral perfusion.

Algorithm development

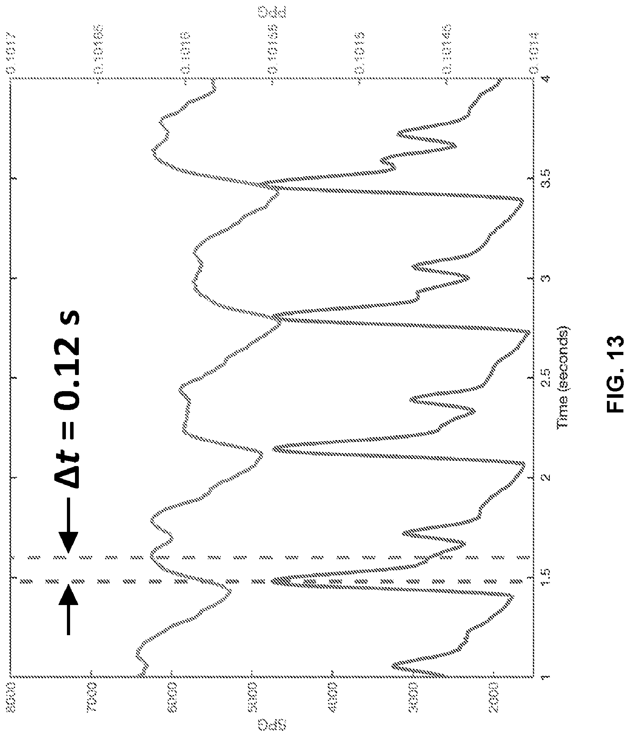

Much information can be extracted from the laser speckle and hemodilution sensors, and this data can be combined in novel algorithms for early detection of hemorrhage, postpartum hemorrhage, treatment response, and general vascular hemodynamic monitoring. For the hemodilution sensor, we anticipate that the ratio of water to hemoglobin will increase as blood loss increases due to water being pulled into the vasculature from the interstitial fluid in the body's attempt to increase circulating blood volume. There will be an AC component and a DC component, similar to pulse oximeters. To extract a vascular hemodilution parameter, we will calculate a ratio of ratios: $R=(AC\lambda water/DC\lambda water)/(AC\lambda hemoglobin/DC\lambda hemoglobin)$. Similar to blood oxygen saturation, we will determine the hemodiltion (HD) according to the equation: $HD = (k1-k2*R)/(h3-k4)*R$, where the k constants are empirically determined for each device during calibration across a variety of hemoglobin concentrations. This sensor can track the effects of various interventions such as fluid supplementation, infusion with packed red blood cells and/or transfusion. This will allow medical providers to identify dangerously low hemoglobin concentrations so they can augment their care to increase hemoglobin concentration. It also has the potential to assess intravascular water content and extravascular water content for edema monitoring during conditions such as preeclampsia. For laser speckle, we can extract laser speckle contrast (k and k', described above), and calculate the mean, standard deviation, peak-to-peak amplitude, pulse variability, frequency content including pulse rate, pulse rise time and fall time, analysis of the data in the frequency domain, including frequency content and harmonics. This can be compared to standard photoplethysmogaph (PPG) data (which can be obtained by simply taking 1 over the natural log of the mean of the intensity image and plotting this value over time) to extract additional information such as the time differences in peak location measured via LSFI and PPG (Figure 13).

Diagnosis could be determined by setting a threshold for a single metric or multiparameter index that would indicate hemorrhage, such as a certain % change from baseline levels, reaching a certain slope, identifying a local minimum or maximum in the derivative or second derivative of the time series data. In settings of hemorrhage, we expect the mean amplitude of the LSFI to decrease, and analysis of the rate of LSFI change over time may help us to discern important parameters such as when the patient is still compensating for blood loss or if their veins have collapsed. You will note in Figure 5 that the LSFI readings plateaued after the vein collapsed, compared with the precipitous decrease prior to vein collapse. This plateau could be an indicator that the patient can no longer compensate and will likely go into hypovolemic shock, for example. Further, the slope of the decrease will likely be an important indicator of the rate of blood loss, as well as the ability of the patient to compensate for blood loss. This also holds true for treatment of blood loss, where we observed a sharp increase in LSFI signal with infusion of crystalloids. It is possible a "compensation challenge" could be performed in patients prior to surgery or labor, either via a mild blood loss or fluid bolus, to identify patients who do not compensate well as these patients could be at higher risk for hypovolemic shock from a relatively small volume of blood loss. Furthermore, more sophisticated algorithms could be developed that incorporate a patient's medical history and variables such as height, weight, BMI, SBP, DBP, PP, mean HR, relevant medications, use of anesthesia and type if applicable, and starting hematocrit or hemoglobin concentration such that the diagnosis algorithms become personalized to each patient for a more accurate determination of early stage hemorrhage as well as treatment monitoring. Once more data is collected, we will employ machine learning techniques to further improve our predictions.

Manufacture of wearable laser speckle sensor design V2

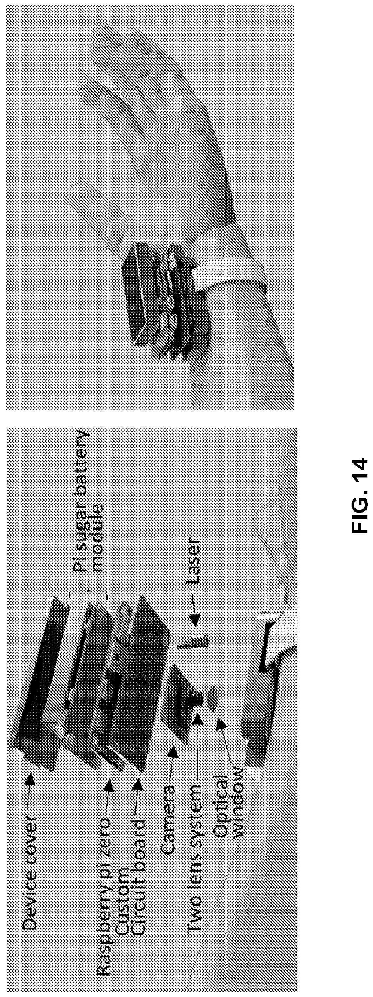

A wearable laser speckle sensor (Figure 14) uses a reflectance mode design (although could be made in transmission) with the same camera, 2-lens system, optical window, and laser as the design illustrated in Figure 4. However, this system is fully wearable, wireless, and powered by a Pi sugar battery module connected to a pi zero 2 W computer board and custom wearable electronics that stabilize the laser output. All components are held in place in a small form factor using custom 3D housings. This new design is showcased in Figure 14.

Summary

The strong correlations observed by our laser speckle sensor in *both* blood loss and crystalloid infusion demonstrate high sensitivity to peripheral perfusion and compensatory mechanisms to stabilize central hemodynamics. These findings have major implications for the ability of this sensor to detect similar changes in trauma patients, surgery patients, and pregnant women to provide an early alert for dangerous blood loss, as well as provide a method to monitor response to treatment and/or interventions that may affect peripheral perfusion and vascular hemodynamics. Further, this device could be used to assess a given patient's ability to compensate for blood volume loss, a notoriously patient-dependent response. This could help with surgical and labor plans to identify high risk individuals that may not have strong compensatory responses and are thus more susceptible to hypovolemic shock.

The above non-limiting example is provided to further illustrate the present disclosure. It should be appreciated by those of skill in the art that the techniques disclosed in the examples represent approaches the inventors have found function well in the practice of the present disclosure, and thus can be considered to constitute examples of modes for its practice. However, those of skill in the art should, in light of the present disclosure, appreciate that many changes can be made in the specific embodiments that are disclosed and still obtain a like or similar result without departing from the spirit and scope of the present disclosure.

**Claims**

1. A system for monitoring hemorrhage in a subject, comprising:

   a. a laser speckle flow index sensor to monitor peripheral perfusion of the subject; and
   b. a multispectral Hb sensor to monitor intravascular hemoglobin concentration of the subject.

2. The system of claim 1, wherein the laser speckle flow index sensor comprises a speckle laser source to illuminate a region of the subject with NIR light and a video camera to obtain laser speckle contrast images of the NIR-illuminated region.

3. The system of any preceding claim, wherein the speckle laser source is a 785 nm laser diode.

4. The system of any preceding claim, wherein the multispectral Hb sensor comprises at least two laser sources to illuminate a region of the subject with light at two different wavelengths, and at least two photodetectors to detect the intensity of light from the illuminated region.

5. The system of any preceding claim, wherein at least two laser sources comprise an 800 nm LED and a 1340 nm LED.

6. The system of any preceding claim, wherein at least two laser sources comprise an 800 nm LED and a 1340 nm LED.

7. The system of any preceding claim, wherein the at least two photodetectors are InGaAs photodiode detectors.

8. The system of any preceding claim, wherein the laser speckle flow index sensor and the multispectral Hb sensor are configured to operate in a transmission mode or a reflection mode.

9. The system of any preceding claim, wherein the system is wearable by the subject.

10. A method of monitoring hemorrhage in a subject, comprising:

   a. providing a device comprising a laser speckle flow index sensor to monitor peripheral perfusion of the subject and a multispectral Hb sensor to monitor intravascular hemoglobin concentration of the subject;
   b. operating the device to monitor peripheral perfusion and intravascular hemoglobin concentration;
   c. indicating a hemorrhage is the peripheral perfusion, the intravascular hemoglobin concentration or both fall below threshold levels.

11. The method of claim 10, wherein the hemorrhage is a Postpartum hemorrhage (PPH).

**FIG. 1A**

FIG. 1B

FIG. 2A

FIG. 2B

FIG. 3

FIG. 4

FIG. 5A

Physiologic Hb Concentrations

- 13.84
- 10.44
- 10.31
- 9.20
- 8.46
- 7.70
- 6.96
- 6.36
- 5.65
- 5.20
- 4.80

FIG. 5B

Spectral ratio vs. concentration

R² = 0.98

FIG. 6

FIG. 7A

FIG. 7B

FIG. 7C

FIG. 8

FIG. 9A

FIG. 9B

FIG. 9C

FIG. 9D

EP 4 640 142 A2

**FIG. 9E**

**FIG. 9F**

G

**Blood Loss After Vein Collapse, R =-0.9**

LSFI = -0.74·Volume+1026.18

LSFI

1560
1540
1520
1500

-740  -720  -700  -680  -660  -640

Blood Loss (mL)

**FIG. 9G**

H

**Blood Loss After Vein Collapse, R =0.99**

LSFI = 1.21·Volume+2389.47

LSFI

1650
1600
1550
1500

-740  -720  -700  -680  -660  -640

Blood Loss (mL)

**FIG. 9H**

EP 4 640 142 A2

I — Crystalloid Only, R = 0.97

LSFI (y-axis): 3000, 2500, 2000, 1500
Crystalloid In (mL) (x-axis): 0, 100, 200, 300, 400

$LSFI = 6.66 \cdot Volume + 6600.47$

J — Crystalloid Only, R = 0.99

LSFI (y-axis): 3000, 2500, 2000, 1500
Crystalloid In (mL) (x-axis): 0, 100, 200, 300, 400

$LSFI = 6.79 \cdot Volume + 6705.81$

FIG. 9I                    FIG. 9J

EP 4 640 142 A2

FIG. 10

**FIG. 11**

**FIG. 12**

**FIG. 13**

EP 4 640 142 A2

FIG. 14

**REFERENCES CITED IN THE DESCRIPTION**

**Patent documents cited in the description**

- US 63162864